# EUROPÄISCHE PATENTSCHRIFT

(11) **EP 4 370 062 B1**
(45) Veröffentlichungstag und Bekanntmachung des Hinweises auf die Patenterteilung: **11.12.2024**
(21) Anmeldenummer: 22750693.8
(22) Anmeldetag: 11.07.2022
(51) Int. Cl.: A61F 2/16

(54) **PUZZLEARTIG AUFGEBAUTE INTRAOKULARLINSE**
INTRAOCULAR LENS WITH A PUZZLE TYPE CONSTRUCTION
LENTILLE INTRAOCULAIRE À UNE CONSTRUCTION DE TYPE PUZZLE

(30) Priorität: 13.07.2021 DE 102021118001
(43) Veröffentlichungstag der Anmeldung: 22.05.2024
(73) Patentinhaber: Carl Zeiss Meditec AG, 07745 Jena (DE)
(72) Erfinder: SCHARSICH, Christina, 14471 Potsdam (DE); BENSAID, Nicolas, 10719 Berlin (DE)
(74) Vertreter: Hofstetter, Schurack & Partner
(86) Internationale Anmeldenummer: PCT/EP2022/069338
(87) Internationale Veröffentlichungsnummer: WO 2023/285392

(56) Entgegenhaltungen:
- US-A- 4 601 722
- US-A- 4 636 210
- US-A- 5 344 448
- US-A1- 2004 148 022

## Beschreibung

### Technisches Gebiet

Ein Aspekt der Erfindung betrifft eine künstliche Intraokularlinse bestehend nur aus einem scheibenförmigen Linsenkörper, der zumindest bereichsweise als Optik der Intraokularlinse ausgebildet ist, wobei der Linsenkörper einen Durchmesser größer oder gleich 8 mm aufweist.

### Stand der Technik

Künstliche Augenlinsen sind als Intraokularlinsen (IOL) bekannt. Es sind hierzu vielfältigste Gestaltungen ermöglicht. So weisen beispielsweise Intraokularlinsen einen optischen Teil auf, an den randseitig zumindest eine Haptik anschließen kann. Mittels dieser Haptik kann die Intraokularlinse im Auge in einem Kapselsack gehalten werden. Die Haptik ist dabei Bestandteil der Intraokularlinse und weist keine optische Abbildungseigenschaft auf, wie dies jedoch der optische Teil hat. Solche Intraokularlinsen weisen einen optischen Teil auf, der einen kleinen Durchmesser, insbesondere in etwa 6 mm oder kleiner, hat. Dadurch können solche Linsen durch einen kleinen Schnitt im Auge implantiert werden. Da der einstückige optische Teil jedoch im Durchmesser im Vergleich zur natürlichen Linse des Auges kleiner ist, benötigt diese Intraokularlinse die Haptik, damit sie im Kapselsack in Position gehalten werden kann. Dennoch können sich diese Intraokularlinsen im Auge positionell verändern, was unerwünscht ist. Unter anderem auch wegen des kleinen Durchmessers kann bei derartigen Intraokularlinsen auch eine negative oder positive Dysphotopsie auftreten. Ebenfalls kann im implantierten Zustand einer solchen Intraokularlinse aufgrund des Schrumpfens des Kapselsacks die Migration von Epithelzellen zum Zentrum des Kapselsacks begünstigt werden, wodurch eine Eintrübung des Sehvermögens auftritt.

Um diese Nachteile zumindest zu reduzieren, sind Intraokularlinsen bekannt, die keine Haptik aufweisen. Sie sind daher nur aus einem optischen Teil gebildet. Bekannt sind solche Intraokularlinsen als WIOL-CF^{®} (The Wichterle intraocular continuous focus lens) Linsen.

### GEAENDERTES BLATT

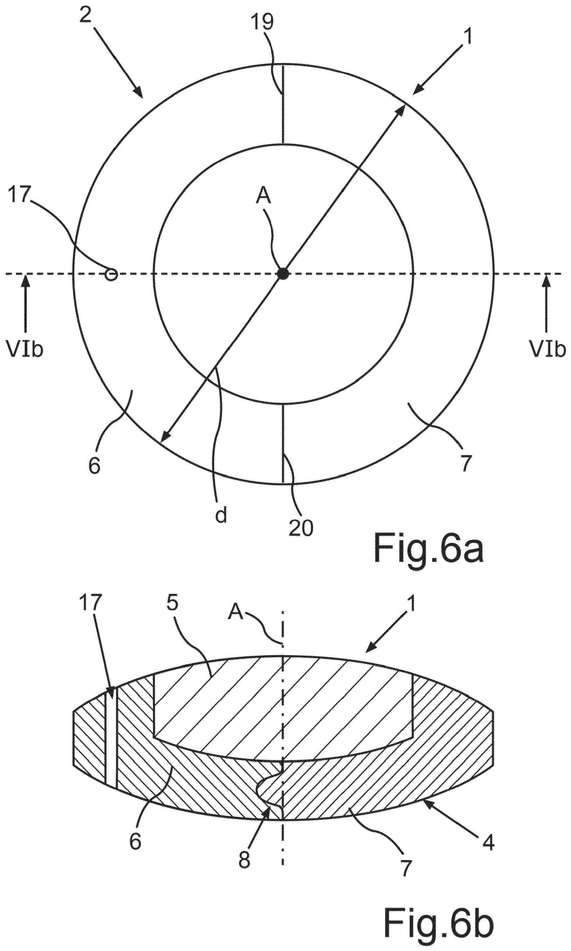

Aus der AU 2013373704 ist eine Intraokularlinse ohne Haptik bekannt. Dort ist eine sehr spezifische Geometrie einer solch spezifischen Intraokularlinse gezeigt. In den Fig. 6A bis 6C sind dort dann Beispiele gezeigt, die eine aus mehreren Bereichen bzw. Lagen gebildete Optik aufweisen. Allerdings erfolgt dort stets die vollständige Fertigung aller Lagen beim grundsätzlichen Herstellen der Intraokularlinse. Des Weiteren sind die Lagen dort praktisch nur "aufeinander gelegt".

Aus der US 9 877 825 B2 ist eine Intraokularlinse mit einem mehrteilig aufgebauten optischen Teil bekannt. Aus der JP 2004- 147 770 A ist eine Intraokularlinse bekannt, bei welcher im optischen Teil zentral liegend Löcher ausgebildet sind.

Aus der US 2004/148022 A1 ist eine mehrteilig aufgebaute und haptiklose Intraokularlinse bekannt.

### Darstellung der Erfindung

Es ist Aufgabe der vorliegenden Erfindung, eine haptiklose Intraokularlinse mit großem Durchmesser des optischen Teils zu schaffen, die durch kleine Schnitte im Auge implantierbar ist.

Diese Aufgabe wird durch eine Intraokularlinse gemäß dem Anspruch 1 gelöst.

Ein Aspekt der Erfindung betrifft eine Intraokularlinse. Die Intraokularlinse besteht nur aus einem scheibenförmigen Linsenkörper. Dieser scheibenförmige Linsenkörper ist zumindest bereichsweise als abbildende Optik der Intraokularlinse ausgebildet. Der Linsenkörper weist einen Durchmesser größer oder gleich 8 mm, insbesondere zwischen 8 mm und 10 mm auf. Der Linsenkörper ist mehrteilig aufgebaut. Er weist ein erstes, vorgefertigtes Linsenkörperteil auf. Der Linsenkörper weist darüber hinaus zumindest ein zum ersten vorgefertigten Linsenkörperteil dazu separates, zweites vorgefertigtes Linsenkörperteil auf. Die zumindest zwei vorgefertigten Linsenkörperteile bilden im zusammengesetzten Zustand den Linsenkörper. Das erste vorgefertigte Linsenkörperteil und das zumindest zweite vorgefertigte Linsenkörperteil sind vor diesem Zusammensetzen jeweils als Formteile ausgebildet, die ihre jeweilige Form beibehalten. Für eine derartige Intraokularlinse ist es nunmehr ermöglicht, einen relativ großen scheibenförmigen Linsenkörper bereitzustellen. Die Intraokularlinse ist diesbezüglich ohne Haptik ausgebildet. Durch einen derartig großen Linsenkörper wird eine Intraokularlinse ermöglicht, die größenmäßig ähnlich oder gleich der natürlichen Linse des Auges ist. Durch diese Größe wird keine Haptik mehr benötigt, um die Intraokularlinse beispielsweise im Kapselsack eines Auges sicher positionieren zu können und diese Position beizubehalten. Darüber hinaus ist es durch die spezifische Ausgestaltung des Linsenkörpers aus mehreren separaten, vorgefertigten Linsenkörperteilen nun auch ermöglicht, eine derartige Intraokularlinse durch einen kleinen Schnitt im Auge einsetzen zu können. Dies wird dadurch ermöglicht, dass die einzelnen Linsenkörperteile einzeln durch diesen kleinen Schnitt im Auge eingeführt werden können. Durch die Ausgestaltung der Linsenkörperteile als ihre jeweilige Form beibehaltende Formteile ist dies ermöglicht. Denn dann kann jedes einzelne Linsenkörperteil zunächst für sich betrachtet hergestellt werden und hierzu auch eine entsprechende Formpräzision aufweisen. Ein Linsenkörperteil, insbesondere alle Linsenkörperteile sind somit auch formfertig bereitgestellt. Dies bedeutet, dass sie für sich alleine betrachtet eine fertige Form aufweisen, die sie vor dem Zusammensetzen mit den anderen Linsenkörperteilen bereits endgültig haben. Ein solches Linsenkörperteil kann dann individuell und unabhängig von den anderen Linsenkörperteilen durch den kleinen Schnitt im Auge ins Auge eingeführt werden. Dies kann mit allen separaten Linsenkörperteilen einzeln erfolgen, die dann gemeinsam den Linsenkörper bilden. Besonders vorteilhaft ist dabei die formerhaltende beziehungsweise die Form beibehaltende Ausgestaltung der Linsenkörperteile. Das bedeutet auch, dass diese Linsenkörperteile für sich betrachtet nach ihrem grundsätzlichen Herstellen die Form selbständig beibehalten, ohne dass sie durch weitere Linsenkörperteile oder anderweitige Komponenten gestützt oder entsprechend umgeben sind. Somit weist jedes Linsenkörperteil auch für sich selbst betrachtet eine Formstabilität auf, die das Erhalten der Basisform des Linsenkörperteils im nicht zusammengesetzten Zustand des Linsenkörpers aufweist. Wie bereits oben erläutert, ermöglicht dies die einzelne Einführung der Linsenkörperteile unabhängig von den anderen Linsenkörperteilen in das Auge. Andererseits wird dabei dann auch durch dieses Einführen die Grundform des Linsenkörperteils beibehalten. Dies bedeutet, dass das grundsätzlich auch elastische und verformbare Material des Linsenkörperteils beim Einführen durch den kleinen Schnitt im Auge elastisch verformt werden kann, jedoch dann im Auge dieses Linsenkörperteil weiterhin seine ursprüngliche Grundform hat beziehungsweise diese unverändert ist. Auch das Einführen in das Auge verändert somit die grundsätzliche Herstellungsform des Linsenkörperteils nicht plastisch. Darüber hinaus hat diese Ausgestaltung von derartigen separaten vorgefertigten Linsenkörperteilen auch den Vorteil, dass das Zusammensetzen des Linsenkörpers sehr präzise erfolgen kann. Dies bedeutet, dass die Linsenkörperteile besonders formschlüssig aneinander anliegen können und somit den gesamten Linsenkörper sehr vorteilhaft bilden. Dies hat wiederum Vorteile bezüglich der optischen Abbildungseigenschaft und der grundsätzlichen mechanischen Stabilität des gesamten Linsenkörpers. Durch die vorgefertigten Linsenkörperteile ist dann auch das Zusammensetzen von den Linsenkörperteilen zu dem Linsenkörper im Auge selbst besonders vorteilhaft ermöglicht. Damit ist es grundsätzlich auch vorteilhaft durch die Erfindung erreicht, dass beim Implantieren der Intraokularlinse in das Auge das eigentliche Zusammensetzen des Linsenkörpers erst im Auge selbst erfolgt. Es werden also nacheinander erst die Linsenkörperteile in das Auge beim Implantieren eingeführt und das Zusammensetzen dieser einzelnen Linsenkörperteile zum gesamten Linsenkörper erfolgt dann erst im Auge selbst. Durch die Formteile ist auch ein für einen Operateur besser erkennbares Zusammensetzungsmuster bzw. ein Zusammensetzungsplan vorgegeben. Denn die Formteile können auch im nicht zusammengesetzten Zustand in ihrer Grundform erkannt werden. Dadurch kann auch erkannt werden, wo und wie ein Linsenkörperteil mit anderen Linsenkörperteilen zusammensetzbar ist. Die Linsenkörperteile sind nicht formsteif beziehungsweise formstarr.

Ein Linsenkörper bzw. ein optischer Teil einer künstlichen Augenlinse ist üblicherweise aus einem Kunststoffmaterial, wie beispielsweise einem Polymermaterial, aufgebaut. Dieser optische Teil weist durch seine Formgebung eine spezifische optische Abbildungseigenschaft auf. Diese optische Abbildungseigenschaft kann refraktiv und/oder diffraktiv sein. Üblicherweise sind in dem Zusammenhang spezifische optische Strukturen in dem optischen Teil ausgebildet oder angeordnet. Damit kann das auf die künstliche Augenlinse aus der Umgebung einfallende Licht durch die optische Abbildungseigenschaft des optischen Teils spezifisch gelenkt werden.

Der Linsenkörper weist zumindest ein Durchgangsloch auf, das sich von einer Vorderseite des Linsenkörpers bis zu einer Rückseite des Linsenkörpers erstreckt. Das diesbezügliche Durchgangsloch ist somit über die gesamte Strecke zwischen der Vorderseite und der Rückseite durchgängig. Insbesondere ist ein derartiges Durchgangsloch in einem Peripheriering des Linsenkörpers ausgebildet. Der Peripheriering kann ein radial äußerer Ringbereich des scheibenförmigen Linsenkörpers sein. Er schließt den Linsenkörper in radialer Richtung nach außen hin ab. Der Peripheriering kann in einem Ausführungsbeispiel ohne optische Abbildungseigenschaft sein. Er kann jedoch auch in einem Ausführungsbeispiel zumindest bereichsweise zur optischen Abbildungseigenschaft des Linsenkörpers beitragen. Durch das oben genannte, zumindest eine Durchgangsloch wird in einem Ausführungsbeispiel ein Spüldurchgangsloch bereitgestellt. Dies bedeutet, dass es bestimmungsgemäß dazu vorgesehen ist, Kammerwasser dort hindurch passieren zu lassen, wenn die Intraokularlinse im Kapselsack implantiert ist. Eine solche Durchgangsbohrung beziehungsweise ein solches Durchgangsloch ist vorteilhaft, da somit die Bildung eines Nachstars (Posterior Capsule Opacification, PCO) verringert oder vermieden werden kann. Vorzugsweise weist der Linsenkörper ein Durchgangsloch auf, mit welcher eine Kanüle gekoppelt werden kann, um eine viskoelastische Substanz (OVD) zwischen einer implantierten Intraokularlinse und einer Rückwand eines Kapselsackes, in welche die Intraokularlinse implantiert ist, abführen zu können. Ein solches Durchgangsloch kann im Peripheriering oder an anderer Stelle des Linsenkörpers angeordnet sein. Darüber hinaus können in einem Ausführungsbeispiel mehrere separate derartiger Durchgangslöcher in dem Linsenkörper ausgebildet sein.

In einem Ausführungsbeispiel weist die Intraokularlinse nur diesen einen einzigen Linsenkörper auf. Dieser ist aus den mehreren Linsenkörperteilen aufgebaut. Der Linsenkörper ist diesbezüglich insbesondere an den Schnittstellen der Linsenkörperteile hohlraumfrei ausgebildet, was für alle Schnittstellen aller Linsenkörperteile gilt.

Der Linsenkörper weist im zusammengesetzten Zustand eine Vorderseite und eine Rückseite aufweisen. Die optische Hauptachse durchdringt mittig die Vorderseite und die Rückseite. Durch die Vorderseite und die Rückseite ist der gesamte Linsenkörper nach vorne und nach hinten begrenzt. Die Vorderseite und die Rückseite definiert auch, insbesondere zumindest bereichsweise, die Geometrie des Linsenkörpers. Die Vorderseite kann plan oder konvex oder konkav gekrümmt sein. In einem Ausführungsbeispiel kann die Rückseite plan oder konvex oder konkav ausgebildet sein. Damit kann der Linsenkörper bikonvex oder bikonkav oder konkav-konvex oder konvex-konkav oder plan-konkav oder konkav-plan oder konvex-plan oder plan-konvex sein.

In einem Ausführungsbeispiel kann auf der diesbezüglich geformten Vorderseite oder der Rückseite in diffraktives Oberflächenprofil und/oder ein torisches Oberflächenprofil zusätzlich aufgebracht sein.

Diesbezüglich gilt also dann auch, dass die Basisfläche der Vorderseite konvex oder konkav ist und/oder die Basisfläche der Rückseite konvex oder konkav ausgebildet ist.

In einem Ausführungsbeispiel sind die Linsenkörperteile an den Bereichen, die zum Verbinden mit dem anderen Linsenkörperteil vorgesehen sind, komplementär geformt. Dies bedeutet auch, dass die Linsenkörperteile in zusammengesetztem Zustand in diesen vorgesehenen Anlagebereichen auch vollständig formschlüssig aneinander anliegen. Damit wird durch die separaten Linsenkörperteile auch ermöglicht, dass der zusammengesetzte Linsenkörper an diesen Stellen hohlraumfrei gebildet werden kann. Auch dies hat besondere Vorteile für die Stabilität des Linsenkörpers und für die optische Abbildungseigenschaft des Linsenkörpers.

In einem Ausführungsbeispiel weisen die zumindest zwei Linsenkörperteile jeweils zumindest ein Verzahnungselement auf. Diese Verzahnungselemente sind zum passgenauen Verzahnen miteinander ausgebildet, so dass die Linsenkörperteile im zusammengesetzten Zustand miteinander verzahnt sind. Dadurch ist lokal eine Verzahnungsvorrichtung gebildet. Damit wird bei diesem Ausführungsbeispiel nicht nur ein bloßes aneinander Anliegen der Linsenkörperteile an ihren Anlagebereichen vorgesehen. Vielmehr ist hier eine mechanische Kopplungsstruktur geschaffen, die die Linsenkörperteile besonders stabil zueinander hält. Diese Verzahnungselemente ermöglichen somit eine Kopplung, mit welcher die Linsenkörperteile quasi ineinander geführt sind. Ein Verzahnungselement ist somit quasi in das andere Verzahnungselement eingeführt. Damit ist auch ein Linsenkörperteil bereichsweise in das andere Linsenkörperteil eingeführt.

Ein Verzahnungselement kann vielfältig ausgestaltet sein. Vorzugsweise ist ein Verzahnungselement ein lokaler Überstand an einem Linsenkörper und das andere Verzahnungselement ist eine lokale Vertiefung an dem anderen Linsenkörperteil. Die beiden Verzahnungselemente sind zum formschlüssigen Kontaktieren über ihre jeweils gesamte Oberfläche ausgebildet. Ein besonders passgenaues, mechanisch stabiles Verbinden dieser Linsenkörperteile ist dadurch ermöglicht.

In einem Ausführungsbeispiel ist ein Verzahnungselement somit ein lokal über eine Begrenzungswand des Linsenkörpers nach außen überstehendes Element. Das komplementäre Verzahnungselement des anderen Linsenkörperteils kann in dem Zusammenhang eine diesbezügliche Vertiefung beziehungsweise Mulde sein, die ausgehend von einer Begrenzungswand dieses anderen Linsenkörperteils sich quasi nach innen erstreckt. Diese nur lokal ausgebildeten Verzahnungselemente ermöglichen eine besonders stabile mechanische Verbindung der Linsenkörperteile. Ein besonders vorteilhaftes lokales Ineinandergreifen der Linsenkörperteile ist dadurch ermöglicht.

In einem Ausführungsbeispiel kann durch diese Verzahnungsvorrichtung mit den Verzahnungselementen ein Verhaken und/oder Verankern der Linsenkörperteile miteinander erreicht werden.

Ein als Überstand ausgebildetes Verzahnungselement kann beispielsweise als eine Spitze, wie beispielsweise als Pyramidenzacken oder als Dreieckzacken oder dergleichen ausgebildet sein. Ebenso ist es möglich, dass ein derartiger Überstand, der ein Verzahnungselement bildet, kugelkopfartig ausgebildet ist. Auch ein pilzartiges Ausführungsbeispiel eines derartigen Verzahnungselements ist möglich. Insbesondere ist das andere Verzahnungselement des anderen Linsenkörperteils, welches zum Koppeln mit diesem Verzahnungselement ausgebildet ist, jeweils in der komplementären Form gestaltet.

Durch eine Verzahnungsvorrichtung, wie sie in den oben genannten Ausführungsbeispielen vielfältig vorgestellt ist, lässt sich auch die positionssichere Anordnung der Linsenkörperteile zueinander verbessern. Es kann zumindest in einer, insbesondere in zumindest zwei Raumrichtungen jeweils eine verbesserte Positionsfixierung der Linsenkörperteile zueinander erreicht werden.

In einem Ausführungsbeispiel ist durch die Verzahnungselemente im verzahnten Zustand eine Halterung der Linsenkörperteile zueinander ausgebildet. Die damit erreichbaren Vorteile wurden bereits oben erläutert.

In einem besonders vorteilhaften Ausführungsbeispiel ist der Linsenkörper durch die Linsenkörperteile als puzzleartiger Gesamtkörper aufgebaut. Insbesondere dann, wenn die Linsenkörperteile Verzahnungselemente aufweisen, ist ein derartiger puzzleartiger Gesamtkörper gebildet. Insbesondere ist der Linsenkörper als 3D-puzzleartiger Gesamtkörper aufgebaut. Die oben genannten Vorteile sind dadurch in besonderem Maße erreicht. Gerade durch die Verzahnungselemente und die individuelle vorgefertigte Form der Linsenkörperteile, die auch formerhaltend bleibt, ist das Zusammensetzen der Linsenkörperteile im Auge für einen Operateur einfach und sicher ermöglicht. Denn durch die individuelle Formgebung und das diesbezügliche Erkennen, weiß der Operateur auch, welches Linsenkörperteil an welcher Stelle beim Zusammensetzen angeordnet werden muss.

Möglich ist es in dem Zusammenhang auch, dass die entsprechenden Linsenkörperteile mit ihren Formgebungen bekannt sind und dem Operateur beispielsweise in einem Operationsmikroskop entsprechend eingeblendet werden. Damit kann der Operateur während des Zusammensetzens der Intraokularlinse im Auge auch unterstützt werden. Diesbezüglich kann optisch in das Mikroskop eingeblendet ein entsprechender Bauplan des Linsenkörpers angezeigt werden und/oder die einzelnen Schritte, welches Linsenkörperteil als nächstes an welche Stelle eines anderen Linsenkörperteils angeordnet werden muss, angezeigt werden.

In einem Ausführungsbeispiel sind die Linsenkörperteile in Richtung einer optischen Hauptachse der Intraokularlinse in Reihe zueinander angeordnet und die Verzahnungselemente sind in Richtung dieser optischen Hauptachse der Intraokularlinse verzahnt. Zusätzlich oder anstatt dazu sind die Linsenkörperteile in Richtung senkrecht zu der optischen Hauptachse der Intraokularlinse in Reihe zueinander angeordnet, und die Verzahnungselemente in Richtung senkrecht zu der optischen Hauptachse der Intraokularlinse verzahnt. Damit ergeben sich vielfältigste Anordnungen der formerhaltenden, vorgefertigten Linsenkörperteile zum diesbezüglichen Aufbau des Gesamtkörpers, nämlich des Linsenkörpers. Damit kann sowohl formspezifisch als auch bezüglich der optischen Abbildungseigenschaft eine hohe Flexibilität und Variabilität bereitgestellt werden, indem diesbezüglich positionell und/oder größenmäßig und/oder materialspezifisch und/oder bezüglich der optischen Abbildungseigenschaft spezifische Linsenkörperteile genutzt und verbaut werden.

Damit wird auch ein besonders vorteilhaftes Baukastensystem für eine derartige spezifische Art einer Intraokularlinse ermöglicht. Denn die einzelnen Linsenkörperteile können dann auch sehr bedarfsgerecht vorgefertigt werden und bleiben auch in dieser Form entsprechend erhalten. Damit kann auch örtlich sehr präzise ein Linsenkörperteil im Linsenkörper verbaut werden. Dadurch ist es auch möglich, dass spezifische Sehfehler durch derartige individuell gestaltete Linsenkörperteile besonders vorteilhaft, auch lokal im gesamten Linsenkörper, kompensiert werden können. Da die Linsenkörperteile diesbezüglich bereits vorgefertigt sind, ist lediglich das Zusammensetzen zu einem Gesamtkörper als Linsenkörper erforderlich. Aufwendige zusätzliche Fertigungsabläufe wie sie bei nicht vorgefertigten Linsenkörperteilen erforderlich sind, müssen daher nicht durchgeführt werden. Dies führt zu wesentlich einfacheren Abläufen beim Zusammensetzen des Linsenkörpers und ermöglicht hier auch, wie bereits oben angesprochen, ein einfaches, schnelleres und dennoch sicheres Zusammenbauen des Linsenkörpers aus den Linsenkörperteilen im Auge selbst.

Vorzugsweise sind die Linsenkörperteile so dimensioniert, dass sie durch einen kleinen Schnitt im Auge eingeführt werden, der eine Länge von kleiner oder gleich 3 mm, insbesondere kleiner oder gleich 2,5 mm, insbesondere kleiner oder gleich 2,0 mm aufweist. Damit können derartige spezifische, haptiklose Intraokularlinsen nunmehr auch durch sehr kleine Schnitte im Auge eingeführt werden.

In einem Ausführungsbeispiel ist der Linsenkörper im zusammengesetzten Zustand der Linsenkörperteile an den Schnittstellen zwischen Linsenkörperteilen hohlraumfrei ausgebildet. Insbesondere gilt dies für alle Schnittstellenbereiche der Linsenkörperteile.

Damit wird bezüglich der Schnittstellen zwischen den Linsenkörperteilen ein massiver Linsenkörper bereitgestellt.

In einem Ausführungsbeispiel können die Linsenkörperteile durch magnetische Haltekraft und/oder durch Klebstoff zueinander beziehungsweise aneinander gehalten sein, um den Linsenkörper aufzubauen. Dies kann zusätzlich zu der mechanischen Verzahnungsvorrichtung, wie sie oben in Ausführungsbeispielen erläutert wurde, vorgesehen sein. Möglich ist es auch, dass Linsenkörperteile nur durch eine derartige Verzahnungsvorrichtung verbunden sind und andere Linsenkörperteile des Linsenkörpers nur durch magnetische Haltekraft und/oder Klebstoff zueinander gehalten sind. Damit können zum Aufbau des Linsenkörpers aus mehreren Linsenkörperteilen auch verschiedene Haltefunktionen realisiert sein. Dies kann vorteilhaft bezüglich der Zugänglichkeit zu einigen Linsenkörperteilen beziehungsweise dem Zusammensetzen mit anderen Linsenkörperteilen sein.

In einem Ausführungsbeispiel sind die Linsenkörperteile nur durch Verzahnungsvorrichtungen miteinander verbunden. Damit werden keine zusätzlichen magnetischen Haltekräfte und/oder zusätzlicher Klebstoff benötigt. Magnetische Haltekräfte können bei Ausführungsbeispielen beispielsweise durch kleine, integrierte Magnete realisiert sein. Auch magnetische Beschichtungen können vorgesehen sein.

In einem Ausführungsbeispiel sind die Linsenkörperteile mit ihrer vorgefertigten Form und somit als vorgefertigte, formerhaltende Formteile so konzipiert, dass sie jeweils nur eineindeutig miteinander verbunden werden können. Somit wird quasi auch eine Montagecodierung für die Linsenkörperteile bereitgestellt und sie können nur in einem einzigen Montageprinzip miteinander verbunden werden. Ein falscher Zusammenbau der Linsenkörperteile kann dadurch vermieden werden. Somit weisen die Linsenkörperteile in einem Ausführungsbeispiel durch ihre individuelle, vorgefertigte Form auch eine Montagecodierung auf.

In einem Ausführungsbeispiel weist der Linsenkörper eine Masse auf, die maximal der Masse der natürlichen Linse entspricht, die durch die Intraokularlinse in dem zu behandelnden Auge ersetzt werden soll. Dadurch können Dezentierungen oder unerwünschte positionelle Ausrichtungen der Intraokularlinse im Auge vermieden werden. Insbesondere weist das Material des Linsenkörpers einen Brechungsindex größer oder gleich 1,43 auf. In einem Ausführungsbeispiel kann der Linsenkörper durch die Art und/oder Position und/oder Anzahl der Linsenkörperteile bezüglich seiner optischen Abbildungseigenschaft individuell gestaltet sein. Damit ist es auch ermöglicht, verschiedene Arten von Linsenkörper und somit verschiedene Arten von Intraokularlinsen abhängig von der Anzahl der Linsenkörperteile und/oder der Position der Linsenkörperteile zueinander und somit der Position der Linsenkörperteile im gesamten Linsenkörper zu erzeugen. In dem Zusammenhang ist es dann auch ermöglicht, dass beispielsweise zumindest ein gleiches Linsenkörperteil für verschiedene Arten zu erzeugender Linsenkörper genutzt werden kann. Damit ist es auch erreicht, dass ein gleiches Linsenkörperteil bei mehreren Arten von herzustellenden Linsenkörpern genutzt werden kann. Damit kann ein Linsenkörperteil auch ein Gleichbauteil sein, das bei verschiedenen Linsenkörpern genutzt werden kann.

Möglich ist es auch, dass ein Linsenkörperteil bezüglich seiner vorgegebenen Grundform gleich ist, jedoch seine materielle Ausgestaltung variieren kann. Damit kann wiederum ein derartiges Linsenkörperteil für verschiedene zu erzeugende Linsenkörper genutzt werden und mit anderen Linsenkörperteilen kombiniert werden. Aufgrund der beispielsweise materiellen Individualität kann dieses jeweilige Linsenkörperteil jedoch dann zu unterschiedlicher Abbildungseigenschaft eines Linsenkörpers beitragen. Damit ist es auch ermöglicht, dass eine bereits implantierte Intraokularlinse durch den Austausch von lediglich beispielsweise einem Linsenkörperteil auch nachträglich noch bezüglich ihrer Eigenschaft verändert beziehungsweise korrigiert werden kann. Insbesondere ist es mit diesem Konzept daher ermöglicht, dass auch nach einer Implantation einer Intraokularlinse, gegebenenfalls auch eine längere Zeit danach, eine Korrektur der Intraokularlinse durchgeführt werden kann, indem nicht alle Linsenkörperteile, die den Linsenkörper bilden, ausgetauscht werden, jedoch zumindest ein Linsenkörperteil ausgetauscht werden kann. Dies ist durch die Ausgestaltung der Linsenkörperteile als grundsätzlich für sich betrachtet formerhaltende Formteile sehr einfach ermöglicht. Der puzzleartige Aufbau des Linsenkörpers ermöglicht diesbezüglich auch relativ einfach einzelne Puzzleteile, nämlich Linsenkörperteile vom Gesamtkörper wieder zu entfernen und durch ein neues oder anderes Linsenkörperteil, welches insbesondere auch die gleiche Grundform aufweist, zu ersetzen.

Grundsätzlich weist die vorgeschlagene Intraokularlinse auch den Vorteil auf, dass sie durch die Linsenkörperteile zusammengesetzt werden kann und diese einzeln, beispielsweise auch durch sehr kleine Schnitte im Auge, die eine Länge kleiner oder gleich 2,0 mm sein können, eingeführt werden können. Insbesondere kann die Anzahl der Linsenkörperteile, die einen Linsenkörper aufbauen sollen, abhängig von spezifischen Parametern variieren. Diese Parameter können beispielsweise die Linsenform beziehungsweise das Linsendesign, die Brechkraft, die Torizität und die Größe des Kapselsacks und des Kleinschnitts im Auge sein.

Weitere Merkmale der Erfindung ergeben sich aus den Ansprüchen, den Figuren und der Figurenbeschreibung. Die vorstehend in der Beschreibung genannten Merkmale und Merkmalskombinationen, sowie die nachfolgend in der Figurenbeschreibung genannten und/oder in den Figuren alleine gezeigten Merkmale und Merkmalskombinationen sind nicht nur in der jeweils angegebenen Kombination, sondern auch in anderen Kombinationen verwendbar, ohne den Rahmen der Erfindung zu verlassen. Es sind somit auch Ausführungen von der Erfindung als umfasst und offenbart anzusehen, die in den Figuren nicht explizit gezeigt und erläutert sind, jedoch durch separierte Merkmalskombinationen aus den erläuterten Ausführungen hervorgehen und erzeugbar sind. Es sind auch Ausführungen und Merkmalskombinationen als offenbart anzusehen, die somit nicht alle Merkmale eines ursprünglich formulierten unabhängigen Anspruchs aufweisen. Es sind darüber hinaus Ausführungen und Merkmalskombinationen, insbesondere durch die oben dargelegten Ausführungen, als offenbart anzusehen, die über die in den Rückbezügen der Ansprüche dargelegten Merkmalskombinationen hinausgehen oder abweichen.

Die in den Unterlagen angegebenen konkreten Werte von Parametern und Angaben zu Verhältnissen von Parametern bzw. Parameterwerten zur Definition von Ausführungsbeispielen der Augenlinse sind auch im Rahmen von Abweichungen, beispielsweise aufgrund von Messfehlern, Systemfehlern, DIN-Toleranzen etc. als vom Rahmen der Erfindung mit umfasst anzusehen, wodurch auch Erläuterungen, die sich auf im Wesentlichen entsprechende Werte und Angaben beziehen darunter zu verstehen sind.

### Kurze Beschreibung der Zeichnungen

Ausführungsbeispiele der Erfindung werden nachfolgend anhand schematischer Zeichnungen näher erläutert. Es zeigen:
- Fig. 1a: eine Draufsicht auf ein Ausführungsbeispiel einer erfindungsgemäßen Intraokularlinse;
- Fig. 1b: eine Schnittdarstellung durch die Intraokularlinse gemäß Fig. 1a;
- Fig. 2a: eine Draufsicht auf ein weiteres Ausführungsbeispiel einer erfindungsgemäßen Intraokularlinse;
- Fig. 2b: eine Schnittdarstellung durch die Intraokularlinse gemäß Fig. 2a;
- Fig. 3a: eine Draufsicht auf ein weiteres Ausführungsbeispiel einer erfindungsgemäßen Intraokularlinse;
- Fig. 3b: eine Schnittdarstellung durch die Intraokularlinse gemäß Fig. 3a;
- Fig. 4: eine Schnittdarstellung durch ein Ausführungsbeispiel einer Verzahnungsvorrichtung zwischen zwei Linsenkörperteilen eines Linsenkörpers einer Intraokularlinse;
- Fig. 5: eine Schnittdarstellung durch ein weiteres Ausführungsbeispiel einer Verzahnungsvorrichtung zwischen zwei Linsenkörperteilen eines Linsenkörpers einer Intraokularlinse;
- Fig. 6a: eine Draufsicht auf eine Intraokularlinse gemäß einem weiteren Ausführungsbeispiel;
- Fig. 6b: eine Schnittdarstellung durch die Intraokularlinse gemäß Fig. 6a;
- Fig. 7: eine Schnittdarstellung durch einen Teilbereich des Ausführungsbeispiels einer Intraokularlinse gemäß der Erfindung, bei welcher zumindest einige Linsenkörperteile durch magnetische Haltekraft und/oder durch Klebstoff miteinander verbunden sind; und
- Fig. 8: eine Schnittdarstellung durch ein weiteres Ausführungsbeispiel einer erfindungsgemäßen Intraokularlinse; und
- Fig. 9: eine Schnittdarstellung durch ein weiteres Ausführungsbeispiel einer erfindungsgemäßen Intraokularlinse.

### Bevorzugte Ausführungsbeispiele der Erfindung

In den Figuren werden gleiche oder funktionsgleiche Elemente mit den gleichen Bezugszeichen versehen.

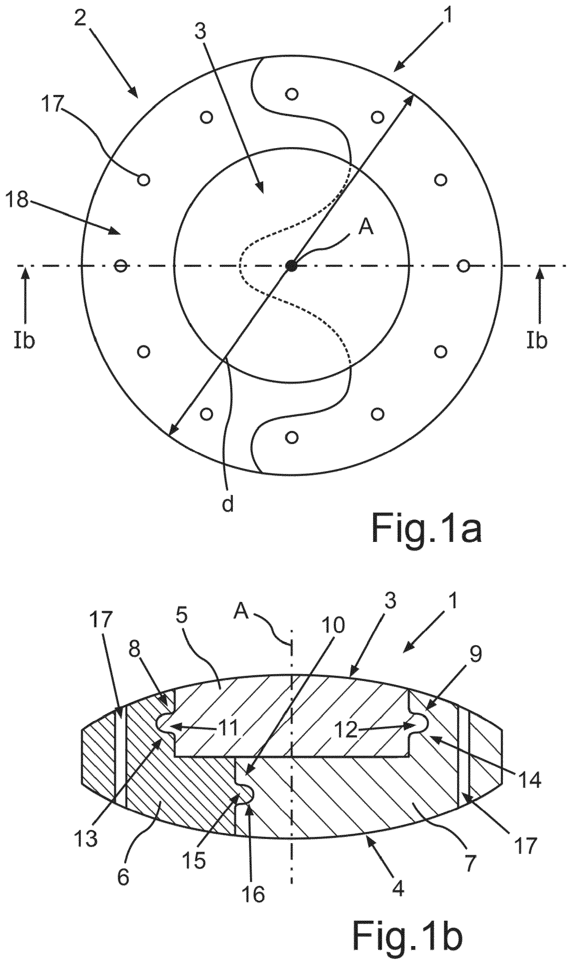

In Fig. 1 ist in einem Ausführungsbeispiel eine Draufsicht auf eine Intraokularlinse 1 gezeigt. Diese künstliche Augenlinse ist bestimmungsgemäß zum Implantieren in ein Auge, insbesondere in einen Kapselsack eines Auges vorgesehen. Die Intraokularlinse 1 besteht hier nur aus einem Linsenkörper 2. Der Linsenkörper 2 ist scheibenförmig. Die Intraokularlinse 1 weist nur diesen einen Linsenkörper 2 auf. Die Intraokularlinse 1 weist keine Haptik auf. Sie hat somit keine Vorrichtung, insbesondere Haltebügel, mit denen sie bestimmungsgemäß im Kapselsack des Auges gehalten wäre. Die Intraokularlinse 1 ist somit auch eine haptiklose Intraokularlinse. Der scheibenförmige Linsenkörper 2 ist flachzylinderartig ausgebildet. Die grundsätzliche Formgebung ist durch eine Vorderseite 3 und/oder eine Rückseite 4 des scheibenförmigen Linsenkörpers 2 definiert. Die Intraokularlinse 1 und somit der scheibenförmige Linsenkörper 2 weisen eine optische Hauptachse A auf. Diese ist in Fig. 1a senkrecht zur Figurenebene orientiert. Sie durchdringt insbesondere mittig die Vorderseite 3 und die Rückseite 4. Im implantierten Zustand ist die Rückseite 4 der Netzhaut des Auges zugewandt und die Vorderseite 3 der Netzhaut des Auges abgewandt.

Die Vorderseite 3 kann mit einer Basisfläche konvex oder konkav gekrümmt sein. Sie kann auch plan sein. Die Rückseite 4 kann mit einer Basisfläche konvex oder konkav sein. Sie kann auch plan sein. Auf diesen Basisflächen kann in einem Ausführungsbeispiel ein diffraktives Oberflächenprofil und/oder ein torisches Oberflächenprofil aufgebracht sein. Der scheibenförmige Linsenkörper 2 ist aus zumindest zwei separaten, vorgefertigten Linsenkörperteilen aufgebaut. Im Ausführungsbeispiel in Fig. 1a und Fig. 1b sind hier als Beispiel drei separate Linsenkörperteile 5, 6 und 7 vorgesehen. Die Linsenkörperteile 5 bis 7 sind vorgefertigte Formteile. Das bedeutet, dass jedes dieser Linsenkörperteile 5 bis 7 für sich betrachtet gefertigt wird und mit einer individuellen Formgebung versehen wird. Ein Linsenkörperteil 5 bis 7 ist nach seiner individuellen Herstellung formerhaltend ausgebildet. Dies bedeutet, dass es auch ohne zusätzliche Stütze oder Hülle oder dergleichen für sich betrachtet seine Form selbständig beibehält. Dennoch ist ein Linsenkörperteil 5 bis 7 aus elastischem Material ausgebildet. Beispielsweise ist dies ein entsprechendes Polymermaterial.

Die Linsenkörperteile 5 bis 7 sind definiert so geformt, dass sie aneinander anliegend positioniert werden können und passgenau zueinander in Verbindung gebracht werden können. Dadurch ist dann der scheibenförmige Linsenkörper 2 aufgebaut. In Fig. 1b ist entlang der Schnittlinie Ib - Ib die Intraokularlinse 1 gemäß Fig. 1a gezeigt. Der zusammengebaute Zustand des scheibenförmigen Linsenkörpers 2 aus den einzelnen Linsenkörperteilen 5 bis 7 ist zu erkennen. An den Anlageflächen und somit an den Schnittstellen zwischen den Linsenkörperteilen 5 bis 7 sind diese insbesondere formschlüssig aneinander anliegend, insbesondere über die gesamte Anlagefläche formschlüssig aneinander anliegend. Dadurch ist der scheibenförmige Linsenkörper 2 an den Schnittstellen der einzelnen Linsenkörperteile 5 bis 7 vollständig hohlraumfrei ausgebildet.

Im gezeigten Ausführungsbeispiel sind die Linsenkörperteile 5 bis 7 jeweils durch lokale individuelle Verzahnungsvorrichtungen 8, 9, 10 mechanisch miteinander verbunden. Diese Verzahnungsvorrichtungen 8 bis 10 sind so gebildet, dass die Linsenkörperteile 5 bis 7 nur lokal ineinandergreifend angeordnet sind. Im gezeigten Ausführungsbeispiel weist das Linsenkörperteil 5 als Überstände ausgebildete Verzahnungselemente 11 und 12 auf. Diese greifen formschlüssig in weitere Verzahnungselemente 13 und 14 ein, die als Vertiefungen in den weiteren Linsenkörperteilen 6 und 7 ausgebildet sind.

Wie darüber hinaus in Fig. 1b auch zu erkennen ist, weisen die Linsenkörperteile 6 und 7 weitere Verzahnungselemente 14 und 15 auf, die auch hier als Überstände und Vertiefungen ausgebildet sind. Dadurch ist auch hier ein entsprechendes ineinandergreifendes Koppeln beziehungsweise eine Verzahnung beziehungsweise eine Verankerung vorgesehen. Insbesondere ist somit jedes Linsenkörperteil 5 bis 7 durch eine entsprechende Verzahnungsvorrichtung an dem daran anliegenden, zumindest einen weiteren Linsenkörperteil 5 bis 7 entsprechend mechanisch gekoppelt. Die Verzahnungsvorrichtungen 8 bis 10 können mit ihren Verzahnungselementen individuell ausgestaltet sein. Hier können als Überstände Pyramidenzacken, Dreieckzacken, Kugelkopfausgestaltungen oder Pilzformausgestaltungen vorgesehen sein. Diese greifen dann in jeweils komplementär geformte Vertiefungen, die die anderen Verzahnungselemente darstellen, ein.

Verzahnungselemente sind allgemein betrachtet insbesondere abrupt und lokal aus der jeweiligen Begrenzungsfläche beziehungsweise Anlagefläche eines Linsenkörperteils hervorstehende oder hineinstehende Überstände oder Vertiefungen.

Wie darüber hinaus in Fig. 1b auch zu erkennen ist, ist ein an der Vorderseite 3 und/oder an der Rückseite 4 jeweils bündiger Übergang zwischen einem entsprechenden Oberflächenbereich der aneinander angrenzenden Linsenkörperteile gebildet. Insbesondere ist somit die gesamte Vorderseite glatt beziehungsweise stufenlos an den Übergängen zwischen Linsenkörperteilen 5 bis 7 ausgebildet. Entsprechendes ist auch an der Rückseite 4 am Übergang zwischen den Linsenkörperteilen 6 und 7 realisiert.

Wie darüber hinaus in Fig. 1b auch zu erkennen ist, ist durch die Verzahnungsvorrichtungen 8 bis 10 jeweils nur lokal ein Überlappen zwischen benachbarten und aneinander anliegenden Linsenkörperteilen 5 bis 7 ausgebildet. So ist beispielsweise in einer Richtung senkrecht zur optischen Hauptachse A ein diesbezügliches Überlappen an der Verzahnungsvorrichtung 8 und an der Verzahnungsvorrichtung 9 und an der Verzahnungsvorrichtung 10 ausgebildet.

Im Ausführungsbeispiel weist die Intraokularlinse 1 zumindest ein Durchgangsloch 17, hier mehrere separate Durchgangslöcher 17 auf. Die Durchgangslöcher 17 sind durchgängig von der Vorderseite 3 bis zur Rückseite 4 ausgebildet. Diese Durchgangslöcher 17 sind bestimmungsgemäß als Durchflusslöcher für Kammerwasser ausgebildet, um eine Nachstarbildung (Posterior Capsule Opacification, PCO) zu verhindern.

In einem Ausführungsbeispiel sind die Durchgangslöcher 17 in radialer Richtung von der optischen Hauptachse A aus betrachtet in einem radial äußeren Ringbereich 18 ausgebildet. Dieser kann ein spezifischer Peripheriering sein. Er kann zur optischen Abbildungseigenschaft des scheibenförmigen Linsenkörpers 2 beitragen. In einem anderen Ausführungsbeispiel ist er ohne optische Abbildungseigenschaft ausgebildet.

Wie in Fig. 1a und 1b zu erkennen ist, ist der zusammengebaute scheibenförmige Linsenkörper 2 als dreidimensionaler puzzleartiger Gesamtkörper aufgebaut. Er weist einen Durchmesser d größer oder gleich 8 mm auf.

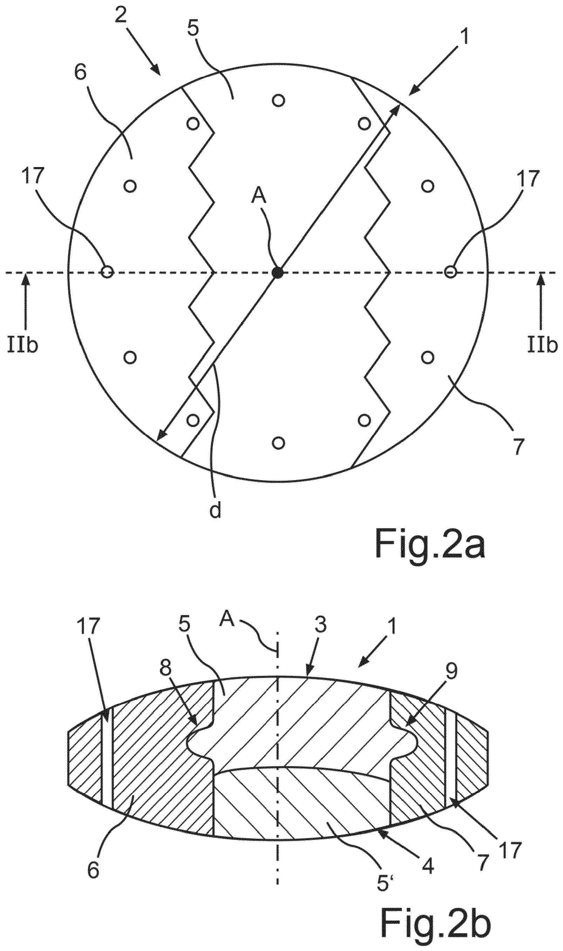

In Fig. 2a ist ein weiteres Ausführungsbeispiel einer Intraokularlinse 1 in einer Draufsicht gezeigt. In Fig. 2b ist diesbezüglich die Schnittdarstellung entlang der Schnittlinie Ilb - Ilb gezeigt. Hier ist lediglich die Formgebung der einzelnen separaten, vorgefertigten Linsenkörperteile 5 bis 7 unterschiedlich zu dem Ausführungsbeispiel in Fig. 1a und 1b. Des Weiteren ist ein weiteres Linsenkörperteil 5' vorhanden.

Wie hier zu erkennen ist, sind hier ebenfalls Verzahnungsvorrichtungen 8 und 9 realisiert. Diese sind zwischen den Linsenkörperteilen 5 und 6 ausgebildet. Zwischen dem Linsenkörperteil 5 und dem Linsenkörperteil 5' ist eine große und nicht nur lokal ausgebildete und sich nicht abrupt erhebende, aber unebene Anlagefläche gebildet. Eine solche Struktur ist keine Verzahnung im Kontext der Erfindung. Es muss hier im Ausführungsbeispiel keine Verzahnungsvorrichtung realisiert sein. Dieses direkte aneinander Anliegen zwischen dem Linsenkörperteil 5 und dem Linsenkörperteil 5' an dieser Anlagefläche kann in einem Ausführungsbeispiel auch durch magnetische Haltekraft und/oder durch Klebstoff realisiert sein. Diese Unebenheit erstreckt sich über den gesamten Anlagebereich zwischen den Linsenkörperteilen 5 und 5'. Es ist daher keine lokale Verzahnung, wie es im Kontext der Erfindung zu verstehen ist.

Grundsätzlich ist es vorteilhaft, wenn zumindest eine mechanische Verbindung zwischen einem Linsenkörperteil und zumindest einem weiteren Linsenkörperteil des scheibenförmigen Linsenkörpers 2 durch zumindest eine Verzahnungsvorrichtung realisiert ist. Grundsätzlich kann in einem Ausführungsbeispiel jegliche diesbezügliche mechanische Kopplung zwischen jeweils aneinander anliegenden Linsenkörperteilen durch zumindest eine Verzahnungsvorrichtung realisiert sein. Weitere Haltevorrichtungen, wie beispielsweise eine magnetische Halterung und/oder eine Halterung durch Klebstoff kann dann entbehrlich sein. In anderen Ausführungsbeispielen kann jedoch zumindest eine weitere Halterung zwischen Linsenkörperteilen vorgesehen sein, die keine Verzahnungsvorrichtung ist.

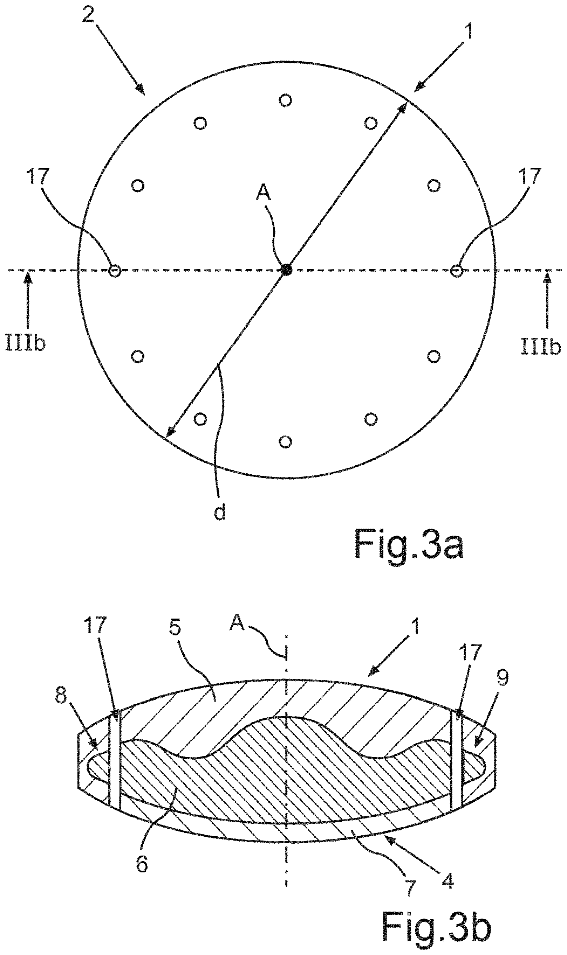

In Fig. 3a ist eine Draufsicht auf ein weiteres Ausführungsbeispiel einer erfindungsgemäßen Intraokularlinse gezeigt. In Fig. 3b ist diesbezüglich die Schnittdarstellung entlang der Schnittlinie Illb - Illb dargestellt. Auch hier sind lediglich beispielsweise zu verstehen drei separate Linsenkörperteile 5 bis 7 vorgesehen, die den Linsenkörper 2 bilden beziehungsweise aus denen der Linsenkörper 2 nur besteht. Daher besteht auch die Intraokularlinse 1 nur aus diesen drei Linsenkörperteilen 5 bis 7.

Bei allen Ausführungsbeispielen können auch mehr oder weniger als drei separate Linsenkörperteile vorgesehen sein, aus denen ein entsprechender Linsenkörper 2 besteht und somit auch die Intraokularlinse 1 dann nur aus diesen mehreren separaten Linsenkörperteilen besteht.

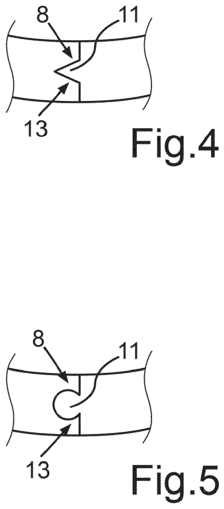

In Fig. 4 ist in einer schematischen Darstellung eine Teilansicht eines Ausführungsbeispiels einer Verzahnungsvorrichtung 8 gezeigt. Diese kann hier als Verzahnungselement 11 einen Pyramidenzacken oder einen Dreieckzacken oder eine sonstige Spitze aufweisen. Die Vertiefung 13 ist diesbezüglich dann komplementär geformt.

In Fig. 5 ist ein weiteres Ausführungsbeispiel für eine Verzahnungsvorrichtung 8 gebildet. Das als Überstand 11 ausgebildete Verzahnungselement kann hier beispielsweise als Kugelkopf oder als Pilzform oder als sonstiger Wulst oder dergleichen ausgebildet sein. Auch hier ist dann das als Vertiefung ausgebildete Verzahnungselement 13 komplementär geformt.

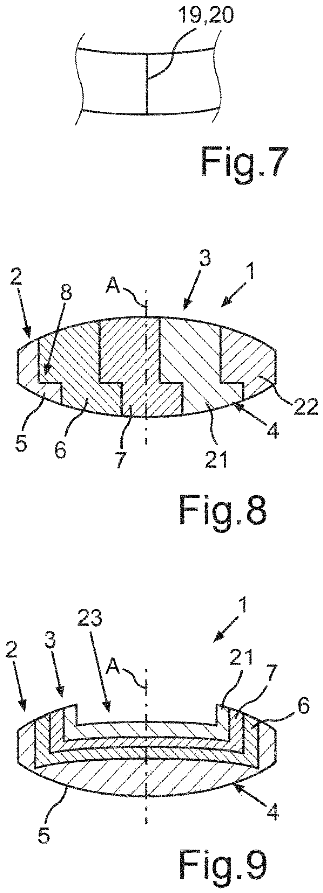

In Fig. 6a ist eine Draufsicht auf ein weiteres Ausführungsbeispiel einer Intraokularlinse 1 mit einem scheibenförmigen Linsenkörper 2 gezeigt. In der Schnittdarstellung in Fig. 6b ist diesbezüglich die Intraokularlinse 1 dargestellt. Auch hier sind lediglich als Ausführungsbeispiel zu verstehen drei separate Linsenkörperteile 5, 6 und 7 vorgesehen. Hier ist diesbezüglich auch wiederum ein symmetrischer Aufbau gebildet, wobei die Linsenkörperteile 6 und 7 im Wesentlichen Halbschalen bilden, in denen das Linsenkörperteil 5 von oben beziehungsweise von vorne mittig eingesetzt angeordnet ist. Mechanische Kopplungen zwischen den Linsenkörperteilen 5 bis 7 können auch wiederum durch zumindest eine Verzahnungsvorrichtung realisiert sein. Zusätzlich können auch an anderen Schnittstellen zwischen Linsenkörperteilen 5 bis 7 andere mechanische Halterungen vorgesehen sein. Beispielsweise können Halterungen 19 realisiert sein, die magnetische Haltekraft aufweisen. Ebenso kann eine diesbezügliche Halterung 20 realisiert sein. Die Halterungen 19 und 20 können in einem anderen Ausführungsbeispiel auch als Klebstoffverbindungen realisiert sein, wie es in Fig. 7 in einem Ausschnitt beispielsweise gezeigt ist.

In Fig. 8 ist in einer Schnittdarstellung ein weiteres Ausführungsbeispiel für eine Intraokularlinse 1 mit nur einem scheibenförmigen Linsenkörper 2 gezeigt. Auch hier sind zwischen mehreren Linsenkörperteilen 5, 6, 7, 21 und 22 individuelle mechanische Halterungen realisiert. Es können auch hier verschiedene oder gleiche entsprechende Halterungen vorgesehen sein. Beispielsweise kann als eine Halterung eine Verzahnungsvorrichtung 8 realisiert sein. Entsprechend kann dies auch an den Schnittstellen der anderen Linsenkörperteile 6, 7, 21 und 22 realisiert sein. Auch hier ist dann ein nur lokales Ineinandergreifen der Linsenkörperteile vorgesehen.

Insbesondere ist dieses Ineinandergreifen bei allen Verzahnungsvorrichtungen relativ abrupt und diesbezüglich beispielsweise gestuft realisiert, wie dies auch bei den anderen Ausführungsbeispielen bisher der Fall war und erläutert wurde. Verzahnungselemente stellen daher eine relativ abrupte Veränderung der Konturenform dar, insbesondere als Ausstülpung oder als Einbuchtung und somit als Überstand oder als Vertiefung. Kontinuierliche Wellenformen oder dergleichen sind daher nicht als derartige lokale und diskrete Verzahnungselemente zu verstehen. Sie sind insbesondere nicht über einen gesamten Anlagebereich zwischen zwei Linsenkörperteilen ausgebildet, sondern nur lokal. Die restliche Fläche eines Anlagebereichs des jeweiligen Linsenkörperteils liegt an dem der anderen Fläche des anderen Linsenkörperteils insbesondere nur an.

In Fig. 9 ist in einem weiteren Ausführungsbeispiel eine Schnittdarstellung durch eine Intraokularlinse 1 mit lediglich einem scheibenförmigen Linsenkörper 2 gezeigt. Hier sind im Querschnitt mehrere, insbesondere U-förmige Linsenkörperteile als separate, vorgefertigte Formteile realisiert, die quasi passgenau ineinander gesetzt sind beziehungsweise aufeinander gesetzt sind. Im Dreidimensionalen sind diese im Querschnitt U-förmigen Linsenkörperteile 5, 6, 7 und 21 topfartig beziehungsweise wannenartig geformt. Auch hier kann dann eine mechanische Verbindung beziehungsweise Halterung wiederum als Verzahnungsvorrichtung ausgebildet sein. Andere Halterungen zwischen anderen Linsenkörperteilen können dann als magnetische Halterung oder als Klebstoffverbindung realisiert sein. Es können auch alle mechanischen Kopplungen wiederum als Verzahnungsvorrichtungen zwischen den einzelnen Linsenkörperteilen 5, 6, 7, 21 realisiert sein. Wie hier zu erkennen ist, ist in einem Ausführungsbeispiel an der Vorderseite 3 hier eine abrupte Stufung des Konturenverlaufs realisiert. Das diesbezüglich vorderste Linsenkörperteil 21 weist einen weiteren Aufnahmebereich 23 auf. Darin kann ein weiteres Linsenkörperteil eingesetzt werden. Dies kann derart geformt sein, dass dann wiederum eine zusammenhängende, insbesondere gleichmäßig verlaufende und an einer entsprechenden Schnittstelle ohne Stufung ausgebildete Vorderseite 3 gebildet wird.

## Patentansprüche

1. Intraokularlinse (1) ohne Haptik, bestehend nur aus einem scheibenförmigen Linsenkörper (2), der zumindest bereichsweise als Optik der Intraokularlinse (1) ausgebildet ist, wobei der Linsenkörper (2) einen Durchmesser (d) größer oder gleich 8 mm aufweist,
wobei der Linsenkörper (2) mehrteilig aufgebaut ist und ein erstes vorgefertigtes Linsenkörperteil (5) und zumindest ein dazu separates, zweites vorgefertigtes Linsenkörperteil (6) aufweist, welche im zusammengesetzten Zustand den Linsenkörper (2) bilden, wobei das erste vorgefertigte Linsenkörperteil (5) und das zweite vorgefertigte Linsenkörperteil (6) vor dem Zusammensetzen jeweils ein ihre jeweilige Form beibehaltendes Formteil sind, wobei der flachzylinderartige Linsenkörper (2) zumindest ein Durchgangsloch (17) aufweist, das sich von einer Vorderseite (3) des Linsenkörpers (2) bis zu einer Rückseite (4) des Linsenkörpers (2) erstreckt und das Durchgangsloch (17) in radialer Richtung von einer optischen Hauptachse (A) der Intraokularlinse (1) aus betrachtet in einem radial äußeren Ringbereich (18) des scheibenförmigen Linsenkörpers (2) ausgebildet ist, wobei der Linsenkörper (2) ansonsten hohlraumfrei ausgebildet ist und die Basisfläche der Vorderseite (3) konvex oder konkav ausgebildet ist und/oder die Basisfläche der Rückseite (4) konvex oder konkav ausgebildet ist.

2. Intraokularlinse (1) nach Anspruch 1,
wobei die beiden Linsenkörperteile (5, 6) jeweils zumindest ein Verzahnungselement (11, 13) aufweisen, die zum passgenauen Verzahnen miteinander ausgebildet sind, so dass die Linsenkörperteile (5, 6) im zusammengesetzten Zustand miteinander verzahnt sind.

3. Intraokularlinse (1) nach Anspruch 2,
wobei ein Verzahnungselement (11) ein lokaler Überstand an einem Linsenkörperteil (5) ist und das andere Verzahnungselement (13) eine lokale Vertiefung an dem anderen Linsenkörperteil (6) ist, wobei die beiden Verzahnungselemente (11, 13) zum formschlüssigen Kontaktieren über ihre jeweils gesamte Oberfläche ausgebildet sind.

4. Intraokularlinse (1) nach Anspruch 2 oder 3,
wobei durch die Verzahnungselemente (11, 13) im verzahnten Zustand eine Halterung der Linsenkörperteile (5, 6) zueinander ausgebildet ist.

5. Intraokularlinse (1) nach einem der vorhergehenden Ansprüche 2 bis 4,
wobei der Linsenkörper (2) durch die Verzahnungselemente (11, 13) als puzzleartiger Gesamtkörper aufgebaut ist.

6. Intraokularlinse (1) nach einem der vorhergehenden Ansprüche,
wobei die Linsenkörperteile (5, 6) in Richtung einer optischen Hauptachse (A) der Intraokularlinse (1) in Reihe zueinander angeordnet sind und die Verzahnungselemente (11, 13) in Richtung der optischen Hauptachse (A) der Intraokularlinse (1) verzahnt sind und/oder in Richtung senkrecht zur optischen Hauptachse (A) verzahnt sind, und/oder die Linsenkörperteile (5, 6) in Richtung senkrecht zu der optischen Hauptachse (A) der Intraokularlinse (1) in Reihe zueinander angeordnet sind und die Verzahnungselemente (11, 13) in Richtung senkrecht zu der optischen Hauptachse (A) der Intraokularlinse () verzahnt sind und/oder in Richtung der optischen Hauptachse (A) verzahnt sind.

7. Intraokularlinse (1) nach einem der vorhergehenden Ansprüche,
wobei das Durchgangsloch (17) ein Spüldurchgangsloch zum Durchleiten von Kammerwasser ist.

8. Intraokularlinse (1) nach einem der vorhergehenden Ansprüche,
wobei die Linsenkörperteile (5, 6) mit magnetischer Haltekraft und/oder mit Klebstoff aneinander gehalten sind.

9. Intraokularlinse (1) nach einem der vorhergehenden Ansprüche,
wobei sich zwischen der Vorderseite (3) und der Rückseite (4) eine im Querschnitt der Intraokularlinse (1) Seitenwand der Intraokularlinse (1) erstreckt, durch welche die flachzylinderartige Form charakterisiert ist.

10. Intraokularlinse (1) nach Anspruch 9,
wobei die Seitenwand einen zur Vorderseite (3) und zur Rückseite (4) unterschiedliche Krümmung aufweist und/oder sich die Seitenwand über ihre gesamte Länge koaxial zur optischen Hauptachse (A) der Intraokularlinse (1) erstreckt.

## Claims

1. Intraocular lens (1) without haptics, consisting solely of a disc-shaped lens body (2), which is designed at least in part as an optical unit of the intraocular lens (1), wherein the lens body (2) has a diameter (d) greater than or equal to 8 mm,
wherein the lens body (2) is constructed in several parts and has a first prefabricated lens body part (5) and at least one separate, second prefabricated lens body part (6), which parts, in the assembled state, form the lens body (2), wherein, prior to their assembly, the first prefabricated lens body part (5) and the second prefabricated lens body part (6) are each a shaped part retaining its respective shape, wherein the flat-cylindrical lens body (2) has at least one through-hole (17) which extends from a front face (3) of the lens body (2) to a rear face (4) of the lens body (2), and the through-hole (17), in a radial direction seen from a main optical axis (A) of the intraocular lens (1), is formed in a radially outer annular region (18) of the disc-shaped lens body (2), wherein the lens body (2) is otherwise formed free of cavities, and the base surface of the front face (3) is convex or concave, and/or the base surface of the rear face (4) is convex or concave.

2. Intraocular lens (1) according to Claim 1,
wherein the two lens body parts (5, 6) each have at least one interlocking element (11, 13), which interlocking elements are designed to interlock exactly with each other, so that the lens body parts (5, 6) are interlocked with each other in the assembled state.

3. Intraocular lens (1) according to Claim 2,
wherein one interlocking element (11) is a local projection on one lens body part (5), and the other interlocking element (13) is a local depression on the other lens body part (6), wherein the two interlocking elements (11, 13) are designed for form-fitting contact over their respective entire surface.

4. Intraocular lens (1) according to Claim 2 or 3,
wherein the interlocking elements (11, 13), in the interlocked state, hold the lens body parts (5, 6) relative to each other.

5. Intraocular lens (1) according to any one of the preceding Claims 2 to 4,
wherein the lens body (2) is constructed, by the interlocking elements (11, 13), as an overall body like a jigsaw puzzle.

6. Intraocular lens (1) according to any one of the preceding claims,
wherein the lens body parts (5, 6) are arranged in series with each other in the direction of a main optical axis (A) of the intraocular lens (1) and the interlocking elements (11, 13) are interlocked in the direction of the main optical axis (A) of the intraocular lens (1) and/or are interlocked in the direction perpendicular to the main optical axis (A), and/or the lens body parts (5, 6) are arranged in series with each other in the direction perpendicular to the main optical axis (A) of the intraocular lens (1) and the interlocking elements (11, 13) are interlocked in the direction perpendicular to the main optical axis (A) of the intraocular lens () and/or are interlocked in the direction of the main optical axis (A) .

7. Intraocular lens (1) according to any one of the preceding claims,
wherein the through-hole (17) is a flushing through-hole for the passage of aqueous humor.

8. Intraocular lens (1) according to any one of the preceding claims,
wherein the lens body parts (5, 6) are held together by magnetic holding force and/or by adhesive.

9. Intraocular lens (1) according to any one of the preceding claims,
wherein a side wall of the intraocular lens (1) extends, in the cross section of the intraocular lens (1), between the front face (3) and the rear face (4) and characterizes the flat-cylindrical shape.

10. Intraocular lens (1) according to Claim 9,
wherein the side wall has a different curvature to the front face (3) and to the rear face (4), and/or the side wall extends over its entire length coaxially with respect to the main optical axis (A) of the intraocular lens (1).

## Revendications

1. Lentille intraoculaire (1) sans haptique, constituée uniquement d'un corps de lentille (2) en forme de disque, qui est réalisé au moins par zones en tant qu'optique de la lentille intraoculaire (1), le corps de lentille (2) présentant un diamètre (d) supérieur ou égal à 8 mm,
le corps de lentille (2) étant construit en plusieurs parties et présentant une première partie de corps de lentille préfabriquée (5) et au moins une deuxième partie de corps de lentille préfabriquée (6) séparée de la première, qui forment le corps de lentille (2) à l'état assemblé, la première partie de corps de lentille préfabriquée (5) et la deuxième partie de corps de lentille préfabriquée (6) étant chacune, avant l'assemblage, une pièce moulée conservant sa forme respective, le corps de lentille de type cylindre plat (2) présentant au moins un trou traversant (17), qui s'étend depuis un côté avant (3) du corps de lentille (2) jusqu'à un côté arrière (4) du corps de lentille (2), et le trou traversant (17) étant réalisé dans une zone annulaire radialement extérieure (18) du corps de lentille en forme de disque (2), vu dans une direction radiale depuis un axe optique principal (A) de la lentille intraoculaire (1), le corps de lentille (2) étant par ailleurs réalisé sans cavité et la surface de base du côté avant (3) étant réalisée convexe ou concave et/ou la surface de base du côté arrière (4) étant réalisée convexe ou concave.

2. Lentille intraoculaire (1) selon la revendication 1,
les deux parties de corps de lentille (5, 6) présentant chacune au moins un élément de denture (11, 13), qui sont réalisés pour s'engrener de manière ajustée l'un avec l'autre, de telle sorte que les parties de corps de lentille (5, 6) sont engrenées l'une avec l'autre à l'état assemblé.

3. Lentille intraoculaire (1) selon la revendication 2,
un élément de denture (11) étant une saillie locale sur une partie de corps de lentille (5) et l'autre élément de denture (13) étant un renfoncement local sur l'autre partie de corps de lentille (6), les deux éléments de denture (11, 13) étant réalisés pour entrer en contact par complémentarité de forme sur toute leur surface respective.

4. Lentille intraoculaire (1) selon la revendication 2 ou 3,
un maintien des parties de corps de lentille (5, 6) l'une par rapport à l'autre étant réalisé par les éléments de denture (11, 13) à l'état engrené.

5. Lentille intraoculaire (1) selon l'une quelconque des revendications 2 à 4 précédentes,
le corps de lentille (2) étant construit par les éléments de denture (11, 13) sous forme de corps global de type puzzle.

6. Lentille intraoculaire (1) selon l'une quelconque des revendications précédentes,
les parties de corps de lentille (5, 6) étant agencées en série les unes par rapport aux autres dans la direction d'un axe optique principal (A) de la lentille intraoculaire (1) et les éléments de denture (11, 13) étant engrenés dans la direction de l'axe optique principal (A) de la lentille intraoculaire (1) et/ou étant engrenés dans la direction perpendiculaire à l'axe optique principal (A), et/ou les parties de corps de lentille (5, 6) étant agencées en série les unes par rapport aux autres dans la direction perpendiculaire à l'axe optique principal (A) de la lentille intraoculaire (1) et les éléments de denture (11, 13) étant engrenés dans la direction perpendiculaire à l'axe optique principal (A) de la lentille intraoculaire () et/ou étant engrenés dans la direction de l'axe optique principal (A) .

7. Lentille intraoculaire (1) selon l'une quelconque des revendications précédentes,
le trou traversant (17) étant un trou traversant d'irrigation pour le passage de l'humeur aqueuse.

8. Lentille intraoculaire (1) selon l'une quelconque des revendications précédentes,
les parties de corps de lentille (5, 6) étant maintenues l'une à l'autre par une force de maintien magnétique et/ou par un adhésif.

9. Lentille intraoculaire (1) selon l'une quelconque des revendications précédentes,
une paroi latérale de la lentille intraoculaire (1) s'étendant entre le côté avant (3) et la face arrière (4) dans la section transversale de la lentille intraoculaire (1), par laquelle la forme de cylindre plat est **caractérisée**.

10. Lentille intraoculaire (1) selon la revendication 9,
la paroi latérale présentant une courbure différente par rapport au côté avant (3) et au côté arrière (4) et/ou la paroi latérale s'étendant sur toute sa longueur coaxialement à l'axe optique principal (A) de la lentille intraoculaire (1).
